# EUROPEAN PATENT APPLICATION

(11) **EP 4 767 956 A1**
(43) Date of publication of application: **01.07.2026**
(21) Application number: 25224217.7
(22) Date of filing: 17.12.2025
(51) Int. Cl.: A61B 17/34, A61B 18/14, A61F 2/95

(54) **STENT DELIVERY SYSTEM FOR PLACING STENT ACROSS ADJACENT TISSUE LAYERS**

(30) Priority: 24.12.2024 KR 20240195536
(71) Applicant: Taewoong Medical Co., Ltd, Gimpo-si, Gyeonggi-do 10022 (KR)
(72) Inventor: LEE, Hyon keun, 10022 Gyeonggi-do (KR); SIM, You Sub, 10022 Gyeonggi-do (KR); KIM, Seung Hyeon, 10022 Gyeonggi-do (KR)
(74) Representative: Porta & Consulenti Associati S.p.A.

(57) **Abstract**

Disclosed is an easy-to-manipulate stent delivery system which facilitates the movement or fixation of an electrocautery tip in adjacent tissue layers, specifically between two adjacent inner cavities in the body, and enables simple deployment of a stent to connect holes of the two adjacent inner cavities created by the electrocautery tip. A first handle, which moves an electrocautery tip, is moved or fixed in a fixed tube by a first locking member which operates semi-automatically. A second handle, which moves an outer tube for deployment of a stent, is moved or fixed on a guide rod of the first handle by a second locking member which is operated by an external pulling force.

## Description

### CROSS REFERENCE TO RELATED APPLICATION

The present application claims priority to Korean Patent Application No. 10-2024-0195536, filed 12 24, 2024, the entire contents of which are incorporated herein for all purposes by this reference.

### BACKGROUND

### Technical Field

The disclosure relates to a stent delivery system for placing a stent across adjacent tissue layers and, more particularly, to a stent delivery system for placing a stent across two adjacent inner cavities to provide a passage for endoscopy, shunt surgery, abscess drainage, and the like.

### Description of the Related Art

When "transluminal" procedures are performed using a stent loaded in a conventional stent delivery system, there is a high probability of leakage of food and digestive fluids, as well as resulting complications, due to repeated exchanges of instruments.

Therefore, a stent delivery system capable of performing electrocautery is now used to enable a one-step procedure without exchanging the instruments as described above. Patent Document 1 also disclosed a device similar to the aforementioned stent delivery system.

As shown in FIG. 1, Patent Document 1 achieved simplified procedures by realizing electrical cauterization performance through a tip 32. However, the problem is that manipulating to place the stent across adjacent tissue layers is difficult.

Specifically, a locking device 20 had to be operated to lock or unlock to move the tip 32 across the adjacent tissue layers, and a locking device 22 had to be operated to lock or unlock to deploy the stent across the adjacent tissue layers.

In other words, Patent Document 1 presented the difficulty of having to operate the locking device 20 and the locking device 22 to lock or unlock, respectively.

Due to the difficulties in manipulation across the adjacent tissue layers for the aforementioned reasons, medical accidents, i.e., errors in electrical cauterization through the tip 32 and errors in deployment of the stent, may occur.

Currently, there is a demand for a stent delivery system which addresses these problems.

The foregoing is intended merely to aid in the understanding of the background of the disclosure, and is not intended to mean that the disclosure falls within the purview of the related art that is already known to those skilled in the art.

### (Documents of Related Art)

(Patent Document 1) United States Patent No. 11766264 (September 26, 2023)

### SUMMARY

Accordingly, the disclosure is intended to provide an easy-to-manipulate stent delivery system which may facilitate the movement or fixation of an electrocautery tip in adjacent tissue layers, specifically between two adjacent inner cavities in the body, and enable simple deployment of a stent to connect holes of the two adjacent inner cavities created by the electrocautery tip.

In order to achieve at least one of the above objectives, the disclosure provide a stent delivery system including: a fixed tube including a first passage provided therein, with a plurality of first catch grooves provided along an outer surface of the fixed tube in a longitudinal direction; a first handle including an inner second passage provided in the first handle and allowing a portion of the fixed tube to be inserted into the inner second passage so that the first handle is movable forward and backward along the outer surface of the fixed tube, a button hole provided in an outer surface of the first handle and connected to the second passage, and a guide rod protruding outward from the outer surface of the first handle in an opposite direction to the fixed tube, wherein a third passage is provided in the guide rod to be connected to the second passage, first and second guide holes are provided on respective sides of an outer surface of the guide rod in the longitudinal direction to be connected to the third passage, a fixing portion is provided at a rear end portion of the third passage, a plug is mounted on the guide rod around the fixing portion, and first and second catch protrusions protrude from respective ends of the second guide hole adjacent to the second passage and the fixing portion, respectively; a first locking member including a seesaw member rotatably mounted in the second passage and having a third catch protrusion and a pressing portion protruding from respective sides and a hinge protruding from an intermediate portion, a spring configured to press the third catch protrusion to be inserted into and caught by the first catch groove; and a button mounted in the button hole to be moved up and down so that when the pressing portion is pressed, the button disengages the third catch protrusion from the first catch groove; a second locking member including a second handle mounted on the outer surface of the guide rod to be movable forward and backward and a moving member inserted into the third passage, wherein the moving member includes first and second guide portions provided on an outer surface of the moving member, extending through the first and second guide holes, respectively, and fixed to the second handle and second and third catch grooves provided on an outer surface of the second guide portion, such that when moved forward or backward by the second handle, the first and second catch protrusions are inserted into and caught by the second and third catch grooves; an outer tube, wherein one side of the outer tube is fixed to the moving member and the opposite side of the outer tube protrudes from the fixed tube; and an inner tube member inserted into an interior of the outer tube, wherein one end of the inner tube member is fixed to the fixing portion, an electrocautery tip is provided on the opposite end of the inner tube member to be caught by the outer tube, the electrocautery tip cauterizing two adjacent inner cavities in a body to form holes, a mounting space is provided behind and adjacent to the electrocautery tip at one opposite side, the stent, which connects the pair of holes, is mounted in the mounting space by being pressed by the outer tube, and a wire connected to the plug is inserted into the inner tube member and connected to the electrocautery tip.

When the button is pressed, the third locking pin disengages from the first locking groove, thereby unlocking the first locking member.

As a result, the first handle, which moves the electrocautery tip, may move in the fixed tube.

When pressure on the button is released, the third locking pin is inserted into and caught by the first locking groove by the spring, thereby locking the first locking member.

Specifically, the first handle, which moves the electrocautery tip, is unable to move in the fixed tube.

In other words, the first handle, which moves the electrocautery tip, has the effect of being moved or fixed in the fixed tube by the first locking member which operates semiautomatically.

For this reason, the disclosure has the effects of providing ease of operation and allowing the electrocautery tip to be easily moved or fixed across adjacent tissue layers, specifically between two adjacent inner cavities within the body.

In addition, the disclosure has the effect of preventing electrocautery errors, i.e., medical accidents which may be caused by the difficulty of operation.

The disclosure has the effect that when the second handle is pulled, the second locking groove disengages from the first locking protrusion, thereby unlocking the second locking member.

That is, the second handle, which moves the outer tube for the deployment of the stent, may move along the guide rod of the first handle.

Continuously pulling the second handle for backward movement causes the third locking groove to be inserted into and caught by the second locking pin, thereby locking the second locking member.

That is, after the deployment of the stent is complete, the second handle, which moves the outer tube, is unable to move along the guide rod of the first handle.

In other words, the second handle, which moves the outer tube for the deployment of the stent, has the effect of being moved or fixed on the guide rod of the first handle by the second locking member which is operated by an external pulling force.

For this reason, the disclosure has the effects of being easy to operate and facilitating the deployment of the stent to connect the holes of the two inner cavities created by the electrocautery tip.

Furthermore, the disclosure has the effect of preventing the deployment of the stent errors, i.e., medical accidents which may be caused by the difficulty of operation.

Because the second and third locking grooves are guided on the rounded guide surfaces of the first and second locking members to be easily inserted into or disengaged from the first and second locking members, the disclosure has the effect that the second handle may be easily released from or fixed to the guide rod of the first handle.

For this reason, the disclosure provides the effect of easily unlocking or locking the second locking member.

The disclosure provides the effect of reliably guiding the rotation of the seesaw member in the holder when the button is pressed or released.

Specifically, the disclosure has the effect of preventing the seesaw member from swaying in the holder due to the external force applied when pressing the button or the elastic force of the restoring spring.

In addition, because the first guide protrusion of the button is guided to the first guide groove of the insertion hole, the disclosure has the effect of preventing the button from wobbling in the insertion hole or the button hole due to external pressure applied to the button or the elasticity of the restoring spring.

For this reason, the disclosure provides the effect of reliably unlocking or locking the first locking member.

The disclosure provides the effect that the male thread portion engages with the female thread portion, thereby pressing the fixed member against the external tube inserted into the fixing hole of the movable member.

In addition, the effect is that the catch portion, which is expanded beyond the diameter of the external tube, is inserted into and caught by the catch hole of the movable member.

Furthermore, the effect is that the outer tube is bonded and fixed to the fixing hole of the moving member by the adhesive.

That is, the effect is that the outer tube is fixed to the moving member.

For this reason, the disclosure has the effect of preventing the outer tube from disengaging from the moving member by allowing the outer tube to move smoothly via the second handle.

The disclosure has the effect of allowing the second handle to be caught by the catch pin during backward movement, thereby causing only a portion, i.e., one flange portion, of the stent, to be deployed.

For this reason, the disclosure has the effect of moving the stent to hold the second handle with the catch pin to prevent the second handle from moving backward along with the guide rod until one flange portion is caught around one hole and the effect of preventing the stent from being deployed at a location other than the intended procedure site.

The disclosure has the effect of guiding the second guide protrusion of the first handle to the second guide groove of the fixed tube to move in the second guide groove.

That is, the effect is that when the first handle moves, the first handle, which moves the electrocautery tip, is prevented from shaking in the fixed tube.

For this reason, the disclosure has the effect of preventing the electrocautery tip from shaking, thereby preventing unintended cauterization of non-target portions and the formation of holes.

### BRIEF DESCRIPTION OF THE DRAWINGS

The above and other objectives, features, and other advantages of the disclosure will be more clearly understood from the following detailed description when taken in conjunction with the accompanying drawings, in which:

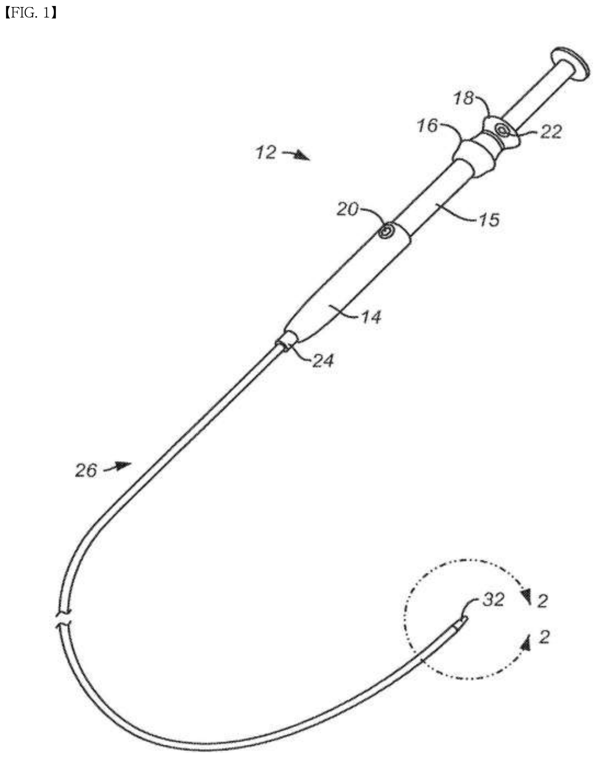

FIG. 1 is a perspective view illustrating a conventional stent delivery system;
FIG. 2 is a perspective view illustrating a stent delivery system according to embodiments of the disclosure;
FIGS. 3 to 5 are plan, side, and bottom views of FIG. 2;
FIG. 6 is a perspective view illustrating the stent delivery system of FIG. 2, from which portions of the first and second handles are removed;
FIG. 7 is an exploded perspective view illustrating the stent delivery system according to embodiments of the disclosure;
FIG. 8 is a cross-sectional view of FIG. 2;
FIGS. 9 to 13 are exploded perspective view of FIG. 8;
FIGS. 14 to 22 are detailed views illustrating the configuration of the stent delivery system according to embodiments of the disclosure; and
FIGS. 23 to 41 illustrate the operational states of the stent delivery system according to embodiments of the disclosure.

### DETAILED DESCRIPTION

Embodiments of the disclosure as described above will be described in detail with reference to the accompanying drawings.

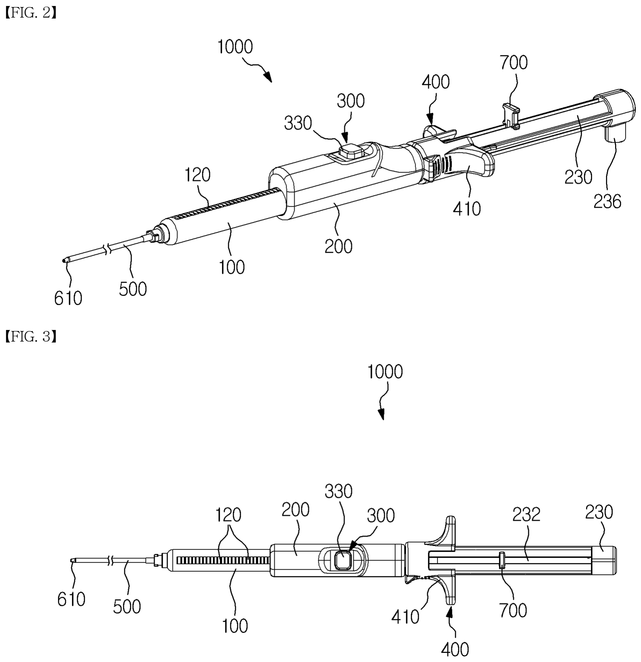

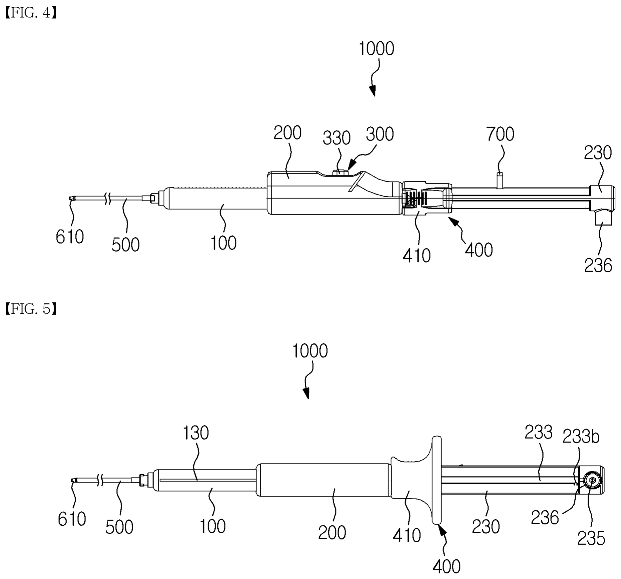

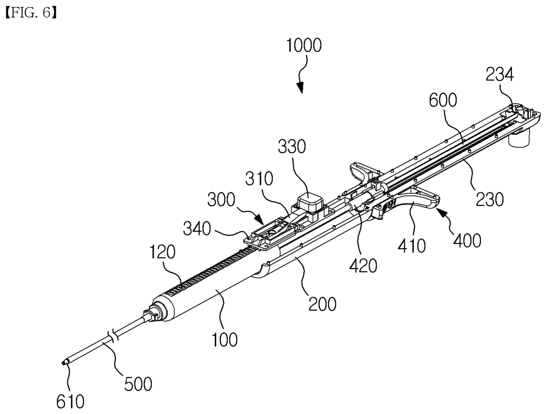

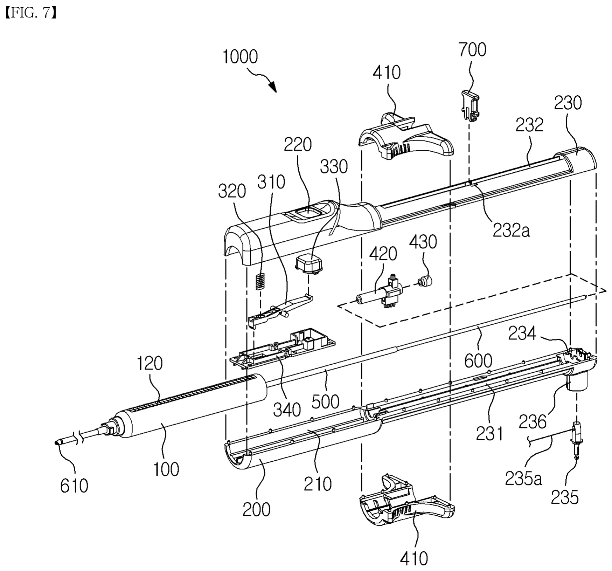

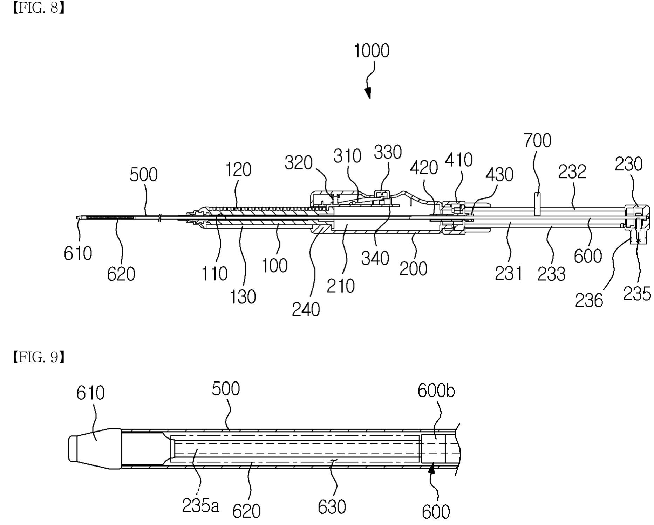

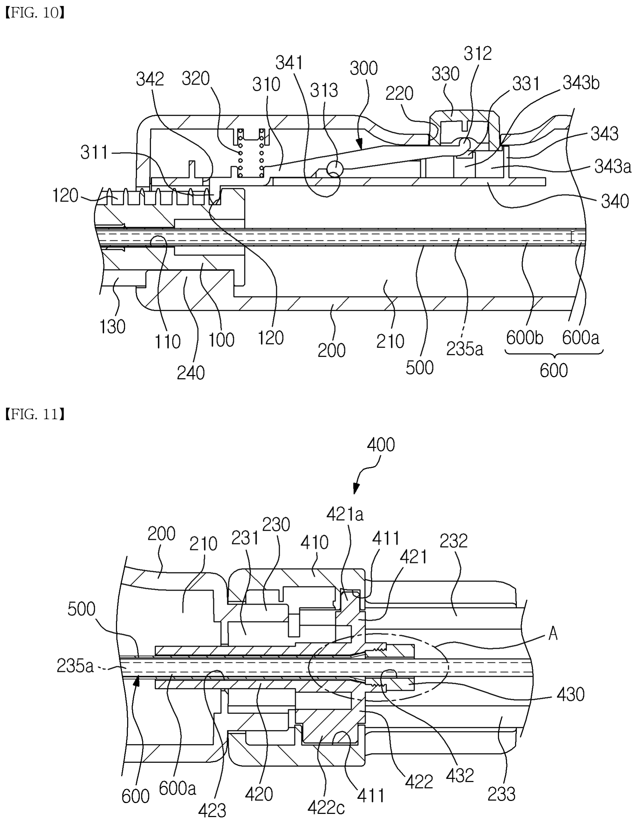

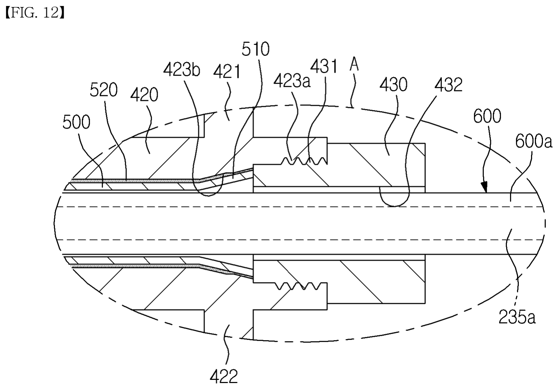

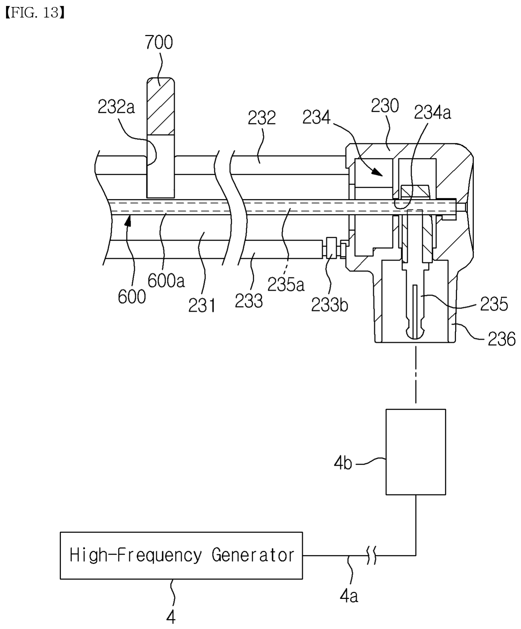

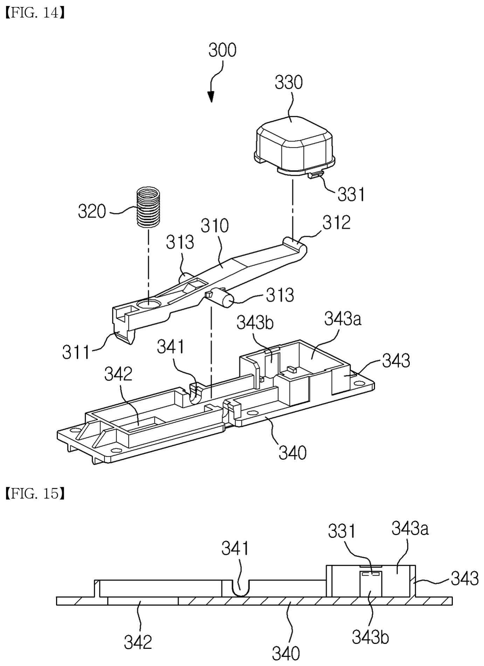

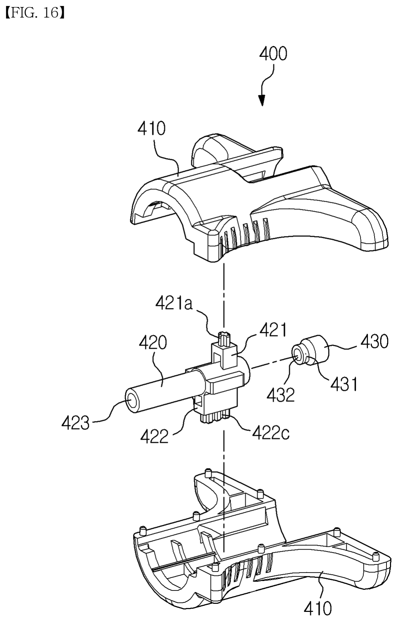

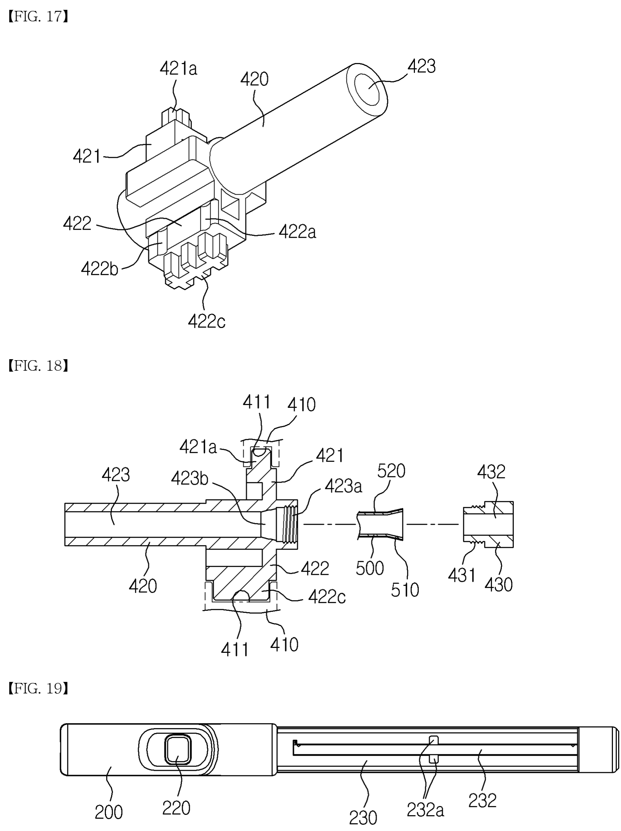

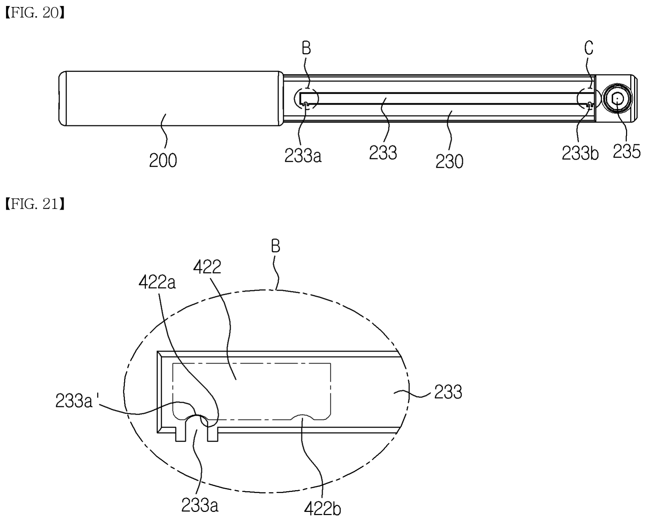

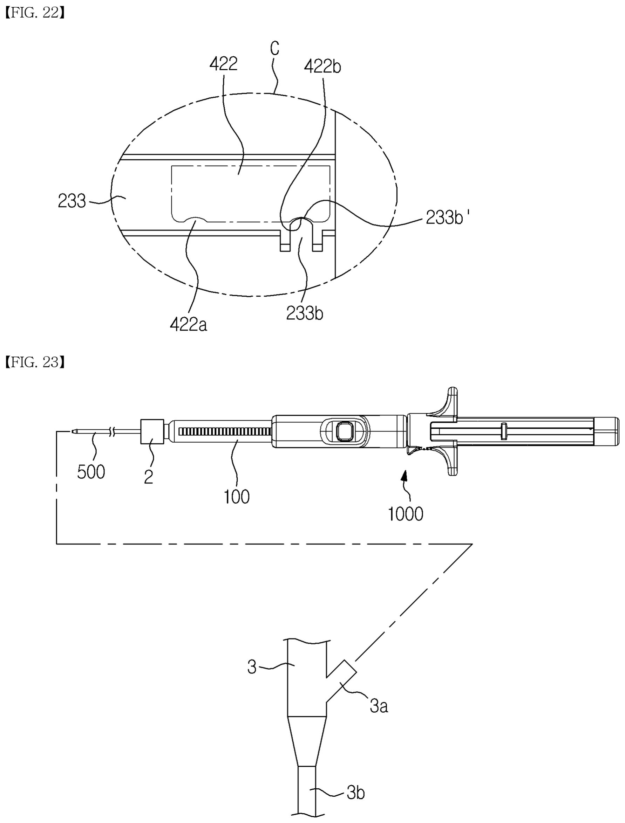

As shown in FIGS. 2 to 41, a stent delivery system 1000 according to embodiments of the disclosure is primarily used when a lesion causing stenosis or obstruction occurs in the bile duct. The stent delivery system 1000 places a stent 620 which connects two adjacent inner cavities 1 in the body, such as the bile duct and stomach, so that digestive enzymes from the liver and the pancreas, or an abscess created at the lesion site, migrate into the stomach.

The stent delivery system 1000 includes a fixed tube 100, a first handle 200, a first locking member 300, a second locking member 400, an outer tube 500, an inner tube member 600, and a locking pin 700.

The fixed tube 100 has a first passage 110 provided therein, with a plurality of first catch grooves 120 provided along the outer surface of the fixed tube 100 in the longitudinal direction.

In this case, a second guide groove 130 is provided on the outer surface of the fixed tube 100 to be elongated in the longitudinal direction away from the first locking groove 120.

The front end of the fixed tube 100 is connected to a connecting portion 3a of an endoscope 3 via a connecting member 2.

The first handle 200 has a second passage 210 which is provided in the first handle 200 and allows a portion of the fixed tube 100 to be inserted thereinto so that the first handle 200 is movable forward and backward on the outer surface of the fixed tube 100. The first handle 200 also has a button hole 220 which is provided in the outer surface of the first handle 200 and connected to the second passage 210. The first handle 200 also has a guide rod 230 protruding outward from the outer surface of the first handle 200 in the opposite direction to the fixed tube 100.

In this case, a second guide pin 240 which is guided to the second guide groove 130 protrudes from the front end portion of the second passage 210, and the guide rod 230 protrudes from the rear portion of the first handle 200. The first handle 200 includes an upper body and a lower body which are coupled to each other.

A third passage 231 is provided in the guide rod 230 to be connected to the second passage 210. First and second guide holes 232 and 233 are provided on respective sides of the outer surface of the guide rod 230 in the longitudinal direction to be connected to the third passage 231. A fixing portion 234 is provided at the rear end portion of the third passage 231. A plug 235 is mounted on the guide rod 230 around the fixing portion 234. First and second catch protrusions 233a and 233b protrude from respective ends of the second guide hole 233 adjacent to the second passage 210 and the fixing portion 234, respectively.

In this case, a cover portion 236 protrudes from the outer surface of the guide rod 230 and covers the plug 235 so that the plug 235 is positioned therein. The plug 235 is linked to a connector 4b of a cable 4a and connected to the high-frequency generator 4.

A rounded guide surface 233a' is provided on the outer surface of the first catch protrusion 233a, and a rounded guide surface 233b' is provided on the outer surface of the second catch protrusion 233b.

An insertion groove 232a is provided in an intermediate portion of the first guide hole 232, and a fixing hole 234a is provided in the fixing portion 234.

The first locking member 300 includes: a seesaw member 310 rotatably mounted in the second passage 210 and including a third catch protrusion 311 and a pressing portion 312 protruding from respective sides and a hinge 313 protruding from an intermediate portion; a spring 320 configured to press the third catch protrusion 311 to be inserted into and caught by the first catch groove 120; and a button 330 mounted in the button hole 220 to be moved up and down so that when the pressing portion 312 is pressed, the button 330 disengages the third catch protrusion 311 from the first catch groove 120.

The first locking member 300 further includes a holder 340 mounted in the second passage 210. The holder 340 has a rotary groove 341 provided in an intermediate portion, into which the hinge 313 is rotatably inserted, a first through-hole 342 provided at one side such that the third catch protrusion 311 which is pressed by the spring 320 extends through the first through-hole 342, and a protrusion 343 provided on the opposite side and having an insertion hole 343a into which the button 330 is inserted to press the pressing portion 312.

In this case, the seesaw member 310 is rotatably mounted in the second passage 210 by the holder 340, and the seesaw member 310 and the holder 340 are positioned over the fixed tube 100.

First guide grooves 343b are provided on respective sides of the insertion hole 343a, and first guide protrusions 331 protrude from respective sides of the outer surface of the button 330 to be guided by the first guide grooves 343b.

The second locking member 400 includes a second handle 410 mounted on the outer surface of the guide rod 230 to be movable forward and backward. The second locking member 400 also includes a moving member 420 which is inserted into the third passage 231. The moving member 420 has first and second guide portions 421 and 422 provided on the outer surface. The first and second guide portions 421 and 422 extend through the first and second guide holes 232 and 233, respectively, and are fixed to the second handle 410. The moving member 420 also has second and third catch grooves 422a and 422b on the outer surface of the second guide portion 422. When moved forward or backward by the second handle 410, the first and second catch protrusions 233a and 233b are inserted into and caught by the second and third catch grooves 422a and 422b.

In this case, the second and third catch grooves 422a and 422b are provided on one side of the second guide member 422 and are rounded to conform to the rounded guide surfaces 233a' and 233b' of the first and second catch protrusions 233a and 233b.

The second handle 410 includes an upper body and a lower body which are coupled to each other, and has insertion grooves 411 provided on the inner upper and lower portions. The first and second guide portions 421 and 422 protrude from the outer surface of the moving member 420 to be guided by the respective first and second guide holes 232 and 233. Insertion protrusions 421a and 422c protrude from surfaces of the first and second guide portions 421 and 422 which face the pair of insertion grooves 411, respectively, to be inserted into the corresponding insertion grooves 411.

One side of the outer tube 500 is fixed to the moving member 420, and the opposite side of the outer tube 500 protrudes from the fixed tube 100.

In this case, the outer tube 500 is inserted into the interior of the connecting portion 3a of the endoscope 3 and into the interior of the tube 3b of the endoscope 3 connected to the connecting portion 3a.

The inner tube member 600 is inserted into the interior of the outer tube 500, wherein one end of the inner tube member 600 is fixed to the fixing portion 234. An electrocautery tip 610 is provided on the opposite end of the inner tube member 600 to be caught by the entrance of the outer tube 500. The electrocautery tip 610 cauterizes two adjacent inner cavities 1 in the body to form holes 1a. At one opposite side, a mounting space 630 is provided behind and adjacent to the electrocautery tip 610. The stent 620, which connects the pair of holes 1a, is mounted in the mounting space 630 by being pressed by the outer tube 500. A wire 235a connected to the plug 235 is inserted into the inner tube member 600 and connected to the electrocautery tip 610.

In this case, the internal tube member 600 is inserted into and fixed in the fixing hole 234a of the fixing portion 234, with a portion of the plug 235 being inserted into the internal tube member 600. The internal tube member 600 is formed from an insulating material.

The electrocautery tip 610 is provided with an insulating member, and a pair of flange portions 621 are provided on respective sides of the stent 620 to be caught around the pair of holes 1a, respectively.

In addition, a guide wire may be inserted into the interior of the inner tube member 600 to provide guidance to the two adjacent inner cavities 1 in the body.

A fixing hole 423 is provided in the inner surface of the moving member 420, into which the outer tube 500 is inserted.

In this case, the outer tube 500 is bonded to the fixing hole 423 using an adhesive 520 such as glue.

A female thread portion 423a is provided on the rear end portion of the fixing hole 423. The second locking member 400 further includes a fixing member 430 having a male thread portion 431 and a second through-hole 432. The male thread portion 431 is provided on the outer surface and engages with the female thread portion 423a. The second through-hole 432 is provided in the central portion and extends through the inner tube member 600. The second locking member 400 presses the outer tube 500 when the male thread portion 431 is engaged with the female thread portion 423a.

In this case, the internal tube member 600 includes a fixed tube 600a and an inner tube 600b. The fixed tube 600a is fixed at one end to the fixing portion 234, with the opposite end being inserted into the interior of the outer tube 500 by passing through the second through-hole 432 of the fixed member 430 and the fixing hole 423 of the movable member 420. The inner tube 600b is connected at one end to the front portion of the fixing tube 600a, with an electrocautery tip 610 being mounted on the opposite end. The inner tube 600b has an assembly space 630 at the opposite side, which is adjacent to and behind the electrocautery tip 610. The inner tube 600b allows the stent 620 to be mounted therein by being pressed by the outer tube 500. The inner tube 600b is inserted into the interior of the outer tube 500.

A portion of the fixing hole 423 adjacent to the female thread portion 423a is configured such that the diameter of the portion increases compared to the fixing hole 423 as the portion is closer to the fixing member 430, thereby forming a catch hole 423b.

A portion of the outer tube 500 adjacent to the female thread portion 423a is configured such that the diameter of the portion increases compared to the outer tube 500 as the portion is closer to the fixing member 430, thereby forming a catch portion 510 which is inserted into and caught by the catch hole 423b.

The stent delivery system 1000 further includes the catch pin 700 which is inserted into the catch groove 232a and catches the second handle 410 when the second handle 410 is moved backward.

As shown in FIGS. 2 to 41, the following description illustrates the operation and effects of the stent delivery system 1000 having the aforementioned configuration according to embodiments of the disclosure.

With the tube 3b of the endoscope 3 inserted to be positioned adjacent to the two inner cavities 1 in the body, the stent delivery system 1000 is positioned around the endoscope 3.

As shown in FIG.S 23 and 24, after the connecting member 2 is mounted on the front portion of the fixed tube 100, the fixed tube 100 is connected to the connecting portion 3a of the endoscope 3 via the connecting member 2 while the outer tube 500 is being inserted into the interiors of the connecting portion 3a and the tube 3b of the endoscope 3.

For this reason, the stent delivery system 1000 is coupled to the endoscope 3.

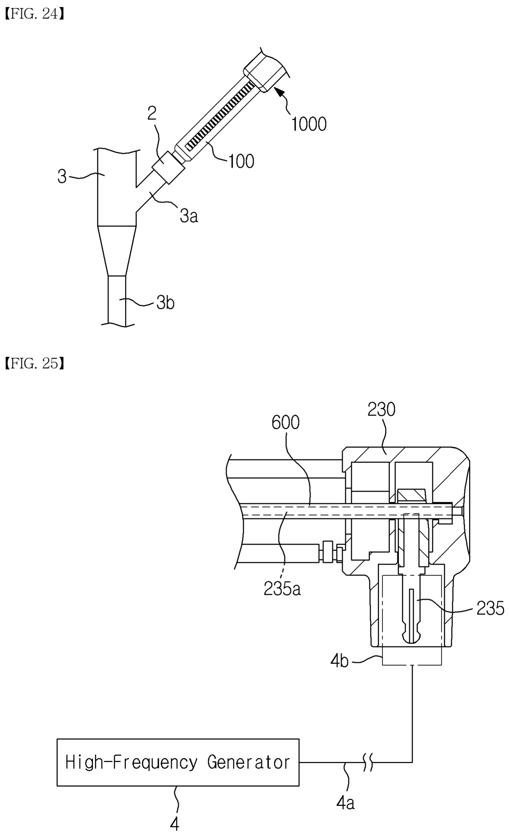

As shown in FIG. 25, the plug 235 is linked to the high-frequency generator 4 and connected to the connector 4b of the cable 4a. The electrocautery tip 610 receives current from the high-frequency generator 4 via the wire 235a, thereby generating high-frequency heat.

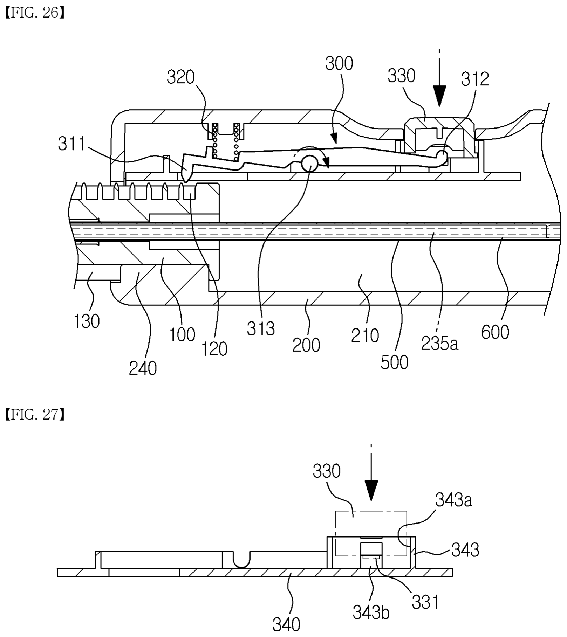

As shown in FIGS. 26 and 27, the first locking member 300 is unlocked to allow the first handle 200 to move on the outer surface of the fixed pipe 100.

More specifically, while the user holds the first handle 200 by hand, the user presses the button 330 with a finger, thereby pressing the pressing portion 312 with the button 330.

Then, the seesaw member 310 rotates about the hinge 313, thereby causing the third catch protrusion 311 to disengage from the first catch groove 120.

In this case, the button 330 is inserted into the insertion hole 343a of the protrusion 343 as the first guide protrusions 331 are guided into the first guide grooves 343b, and the spring 320 is compressed at one side of the seesaw member 310 where the third catch protrusion 311 is provided.

For this reason, the first handle 200 is free from the fixed tube 100 due to the unlocking of the first locking member 300.

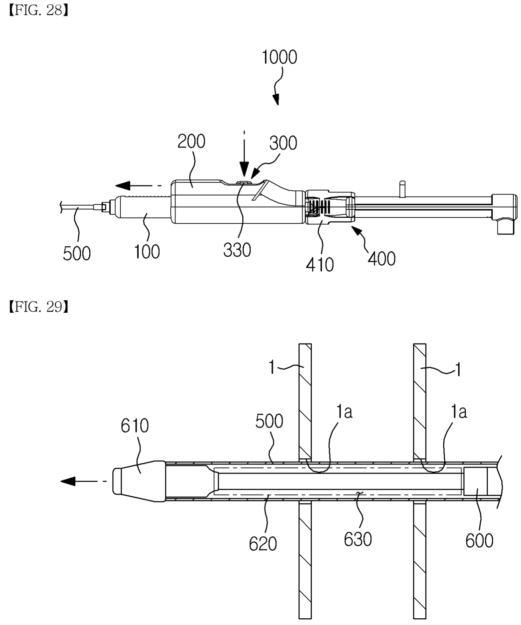

As shown in FIG. 28, the first handle 200 is moved forward along the outer surface of the fixed tube 100.

In this case, the second guide protrusion 240 of the first handle 200 is guided into the second guide groove 130 of the fixed tube 100.

Then, the second handle 410 is fixed to the guide rod 230 of the first handle 200, with the first catch protrusion 233a being inserted into and caught by the second catch groove 422a of the moving member 420. Consequently, the second handle 410 moves forward together with the first handle 200.

The outer tube 500 is also bonded and fixed to the fixing hole 423 using the adhesive 520. The outer tube 500 is fixed to the moving member 420, with the catch portion 510 being pressed against the fixing member 430 while being inserted into and caught by the catch hole 423b. Consequently, the outer tube 500 moves forward together with the second handle 410.

The inner tube member 600 is also fixed to the fixing portion 234 of the guide rod 230, so that the inner tube member 600 moves forward together with the first handle 200.

As shown in FIG. 29, as the electrocautery tip 610 moves together with the first handle 200, the electrocautery tip 610 cauterizes and incises the adjacent two inner cavities 1 in the body to form the holes 1a.

That is, the respective holes 1a are formed by burning and perforating the two adjacent inner cavities 1 with high-frequency heat generated by electrocautery tip 610.

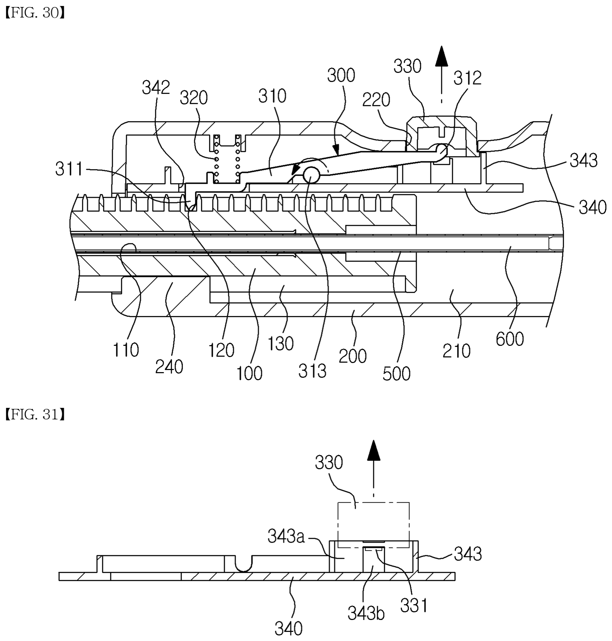

As shown in FIGS. 30 and 31, after the holes 1a are formed, the first locking member 300 is locked to prevent the first handle 200 from moving on the outer surface of the fixed tube 100.

More specifically, the pressing action of the button 330 on the pressing portion 312 is released.

Then, the spring 320 causes the seesaw member 310 to rotate about the hinge 313 while restoring the original state from the compressed state. The third catch protrusion 311 is pressed against the spring 320 and is inserted into and caught by the first catch groove 120.

In this case, as the first guide protrusions 331 are guided to the first guide grooves 343b, the button 330 protrudes from the insertion hole 343a of the protrusion 343 and the button hole 220.

For this reason, the first handle 200 is fixed to the fixed pipe 100 due to the locking of the first locking member 300.

In addition, the plug 235 and the connector 4b of the cable 4a are disconnected, or the operation of the high-frequency generator 4 is stopped.

Then, high-frequency heat is not generated any longer at the electrocautery tip 610.

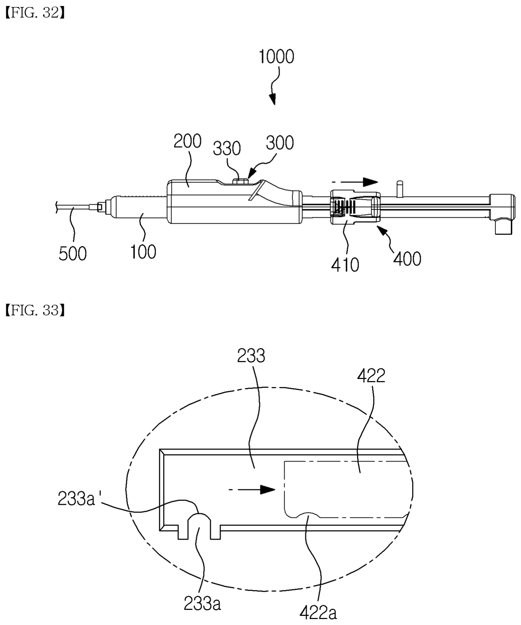

As shown in FIGS. 32 and 33, the second locking member 400 is unlocked to allow the second handle 410 to move along the outer surface of the guide rod 230.

More specifically, the second handle 410 is pulled manually by the user to move backward along the outer surface of the guide rod 230, thereby disengaging the second catch groove 422a of the moving member 420 from the first catch protrusion 233a.

In this case, the second catch groove 422a of the movable member 420 is guided by the rounded guide surface 233a' of the first catch protrusion 233a to be disengaged easily.

For this reason, the second locking member 400 is unlocked by an external force pulling the second handle 410, and the second handle 410 is free from the guide rod 230 due to the unlocking of the second locking member 400.

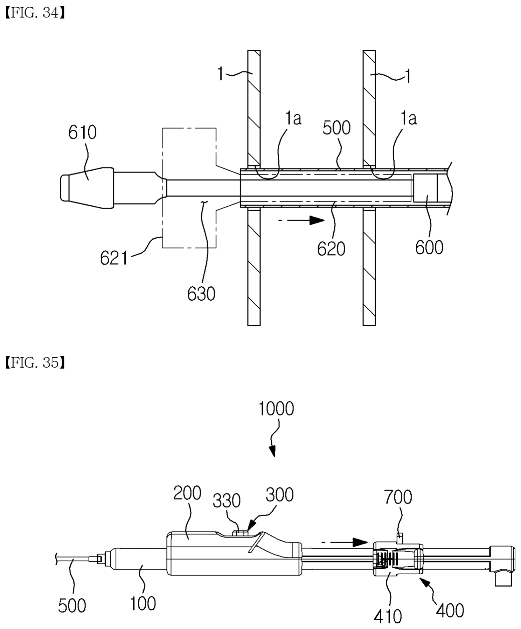

As shown in FIGS. 34 and 35, the moving member 420 retracts together with the second handle 410 as the first and second guide portions 421 and 422 are guided to the first and second guide holes 232 and 233 of the guide rod 230, respectively.

Then, the outer tube 500 fixed to the moving member 420 moves backward together with the second handle 410, and the stent 620, which has been compressed in the mounting space 630 of the inner tube member 600 by the outer tube 500, partially deploys (or expands) to restore the original state due to the backward movement of the outer tube 500.

At this point, the second handle 410 is caught by the catch pin 700 and the backward movement is stopped, and only one of the pair of flange portions 621 is disengaged from the mounting space 630, thereby deploying (or expanding) the stent 620.

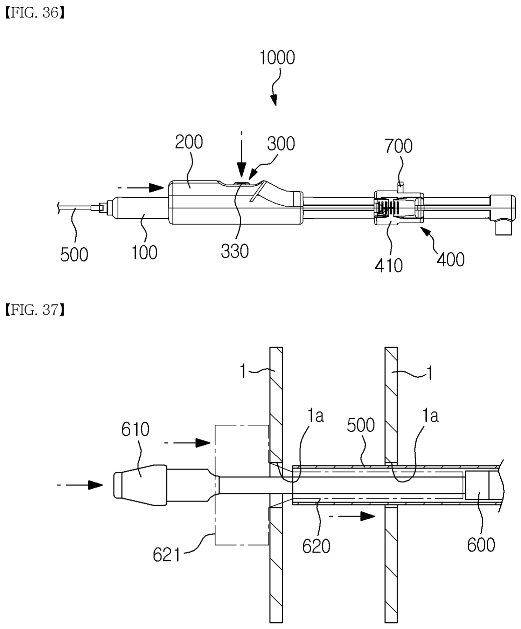

As shown in FIGS. 36 and 37, the user unlocks the first locking member 300 as described above by pressing the button 330 to. Thereafter, the user grasps the second handle 410 with one hand and fixes the second handle 410 to the guide rod 230. In this state, the user pulls the first handle 200 with the other hand to move the first handle 200 backward along the outer surface of the fixed tube 100.

Then, the inner tube member 600 and the outer tube 500, which are fixed to the first and second handles 200 and 410, respectively, move backward along the pair of holes 1a. The stent 620, a portion of which has been compressed to the mounting space 630 of the inner tube member 600 by the outer tube 500, also moves backward.

In this case, the flange portion 621 of the stent 620, which has been disengaged from the mounting space 630, moves toward the hole 1a.

Then, when the flange portion 621 is caught around the holes 1a, the user locks the first locking member 300 in the aforementioned manner by releasing pressure on the button 330, and removes the hand from the second handle 410.

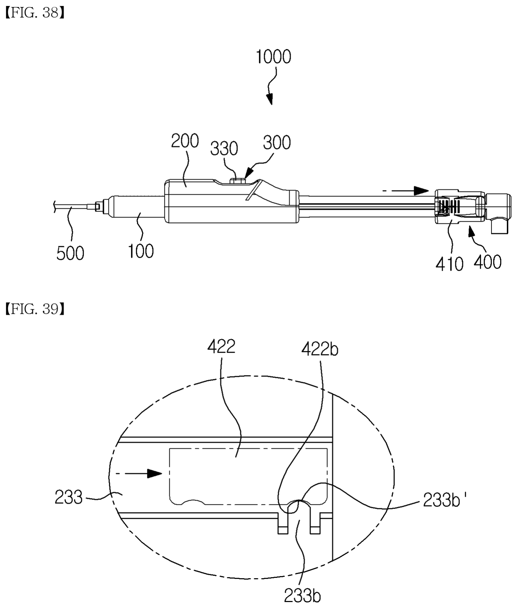

As shown in FIGS. 38 and 39, after disengaging the catch pin 700 from the catch groove 232a, manually pulling the second handle 410 by the user moves the second handle 410 backward along the outer surface of the guide rod 230.

Then, the third catch groove 422b of the moving member 420 is inserted into and caught by the second catch protrusion 233b, thereby preventing the second handle 410 from moving along the outer surface of the guide rod 230.

In this case, the third catch groove 422b of the moving member 420 is guided by the rounded guide surface 233b' of the second catch protrusion 233b to be easily inserted into and caught by the second catch protrusion 233b.

For this reason, as the second locking member 400 is locked by the external force pulling the second handle 410, the second handle 410 is fixed to the guide rod 230.

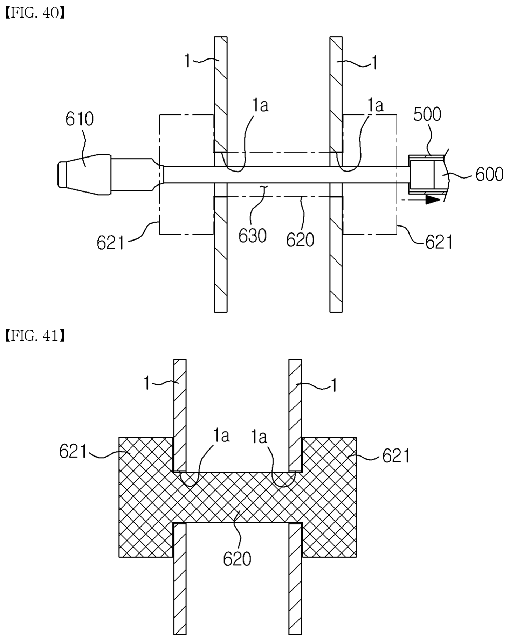

As shown in FIGS. 40 and 41, the stent 600 is fully deployed or expanded) to the original state and completely disengaged from the mounting space 630 of the inner tube member 600, and the pair of flange portions 621 are caught around the pair of holes 1a.

For this reason, the stent 600 connects the holes 1a of the two adjacent inner cavities 1 in the body to each other.

Upon completion of the procedures using the stent 600, the stent delivery system 1000 of the disclosure is detached from the endoscope 3, and the outer tube 500 and the inner tube member 600 are removed from the body.

Although the example embodiments of the disclosure have been described for illustrative purposes, the disclosure is not limited to the aforementioned embodiments. Rather, those skilled in the art will appreciate that various modifications and alterations are possible, without departing from the spirit of the disclosure.

## Claims

1. A stent delivery system comprising:
a fixed tube (100) comprising a first passage (110) provided therein, with a plurality of first catch grooves (120) provided along an outer surface of the fixed tube (100) in a longitudinal direction;
a first handle (200) comprising an inner second passage (210) provided in the first handle (200) and allowing a portion of the fixed tube (100) to be inserted into the inner second passage (210) so that the first handle (200) is movable forward and backward along the outer surface of the fixed tube (100), a button hole (220) provided in an outer surface of the first handle (200) and connected to the second passage (210), and a guide rod (230) protruding outward from the outer surface of the first handle (200) in an opposite direction to the fixed tube (100), wherein a third passage (231) is provided in the guide rod (230) to be connected to the second passage (210), first and second guide holes (232, 233) are provided on respective sides of an outer surface of the guide rod (230) in the longitudinal direction to be connected to the third passage (231), a fixing portion (234) is provided at a rear end portion of the third passage (231), a plug (235) is mounted on the guide rod (230) around the fixing portion (234), and first and second catch protrusions (233a, 233b) protrude from respective ends of the second guide hole (233) adjacent to the second passage (210) and the fixing portion (234), respectively;
a first locking member (300) comprising a seesaw member (310) rotatably mounted in the second passage (210) and having a third catch protrusion (311) and a pressing portion (312) protruding from respective sides and a hinge (313) protruding from an intermediate portion, a spring (320) configured to press the third catch protrusion (311) to be inserted into and caught by the first catch groove (120), and a button (330) mounted in the button hole (220) to be moved up and down so that when the pressing portion (312) is pressed, the button (330) disengages the third catch protrusion (311) from the first catch groove (120);
a second locking member (400) comprising a second handle (410) mounted on the outer surface of the guide rod (230) to be movable forward and backward and a moving member (420) inserted into the third passage (231), wherein the moving member (420) comprises first and second guide portions (232, 233) provided on an outer surface of the moving member (420), extending through the first and second guide holes (232, 233), respectively, and fixed to the second handle (410) and second and third catch grooves (422a, 422b) provided on an outer surface of the second guide portion (422), such that when moved forward or backward by the second handle (410), the first and second catch protrusions (233a, 233b) are inserted into and caught by the second and third catch grooves (422a, 422b);
an outer tube (500), wherein one side of the outer tube (500) is fixed to the moving member (420) and the opposite side of the outer tube (500) protrudes from the fixed tube (100); and
an inner tube member (600) inserted into an interior of the outer tube (500), wherein one end of the inner tube member (600) is fixed to the fixing portion (234), an electrocautery tip (610) is provided on the opposite end of the inner tube member (600) to be caught by the outer tube (500), the electrocautery tip (610) cauterizing two adjacent inner cavities (1) in a body to form holes (1a) , a mounting space (630) is provided behind and adjacent to the electrocautery tip (610) at one opposite side, a stent (620), which connects the pair of holes (1a), is mounted in the mounting space (630) by being pressed by the outer tube (500), and a wire (235a) connected to the plug (235) is inserted into the inner tube member (600) and connected to the electrocautery tip (610).

2. The stent delivery system of claim 1, wherein a rounded guide surface (233a') is provided on an outer surface of the first catch protrusion (233a), and a rounded guide surface (233b') is provided on an outer surface of the second catch protrusion (233b).

3. The stent delivery system of claim 1 or 2, wherein the first locking member (300) further comprises a holder (340) mounted in the second passage (210), wherein the holder (340) has a rotary groove (341) provided in an intermediate portion, into which the hinge (313) is rotatably inserted, a first through-hole (342) provided at one side such that the third catch protrusion (311) which is pressed by the spring (320) extends through the first through-hole (342), and a protrusion (343) provided on the opposite side and having an insertion hole (343a) into which the button (330) is inserted to press the pressing portion (312).

4. The stent delivery system of claim 3, wherein a first guide groove (343b) is provided on the insertion hole (343a), and a first guide protrusion (331) protrudes from an outer surface of the button (330) to be guided by the first guide groove (343b).

5. The stent delivery system of any of claims 1-4, wherein a fixing hole (423) is provided in an inner surface of the moving member (420), into which the outer tube (500) is inserted,
a female thread portion (423a) is provided on a rear end portion of the fixing hole (423), and
the second locking member (400) further comprises a fixing member (430) having a male thread portion (431) provided on an outer surface to engage with the female thread portion (423a) and a second through-hole (432) provided in a central portion and extends through the inner tube member (600).

6. The stent delivery system of claim 5, wherein a portion of the fixing hole (423) adjacent to the female thread portion (423a) is configured such that the diameter of the portion increases compared to the fixing hole (423) as the portion is closer to the fixing member (430), thereby forming a catch hole (423b), and
a portion of the outer tube (500) adjacent to the female thread portion (423a) is configured such that the diameter of the portion increases compared to the outer tube (500) as the portion is closer to the fixing member (430), thereby forming a catch portion (510) which is inserted into and caught by the catch hole (423b) .

7. The stent delivery system of claim 5 or 6, wherein the outer tube (500) is bonded to the fixing hole (423) using an adhesive (520).

8. The stent delivery system of any of claims 1-7, wherein an insertion groove (232a) is provided in the first guide hole (232),
the stent delivery system further comprising a catch pin (700) which is inserted into the insertion groove (232a) and catches the second handle (410) when the second handle (410) is moved backward.

9. The stent delivery system of any of claims 1-8, wherein a second guide groove (130) is provided on the outer surface of the fixed tube (100) to be elongated in the longitudinal direction away from the first locking groove (120), and
a second guide pin (240) is provided in the second passage (210) to be guided to the second guide groove (130).
